Europäisches Patentamt

European Patent Office

Office européen des brevets

(11) Veröffentlichungsnummer: **0 368 223**
**A2**

(12) **EUROPÄISCHE PATENTANMELDUNG**

(21) Anmeldenummer: **89120550.2**

(22) Anmeldetag: **07.11.89**

(51) Int. Cl.⁵: **A61K 31/19**

(30) Priorität: **10.11.88 DE 3838068**

(43) Veröffentlichungstag der Anmeldung:
**16.05.90 Patentblatt 90/20**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hucho, Ferdinand, Prof. Dr.**
**Giesebrechtstrasse 19**
**D-1000 Berlin 12(DE)**
Erfinder: **Pribilla, Iris, Dr.**
**Im Bildsacker 33**
**D-6903 Neckargemünd-Dilsberg(DE)**
Erfinder: **Schiebler, Werner, Dr.**
**Ziegenhainer Strasse 87**
**D-6000 Frankfurt am Main 50(DE)**
Erfinder: **Tripier, Dominique, Dr.**
**Im Kirschgarten 16**
**D-6239 Eppstein/Taunus(DE)**
Erfinder: **Kogler, Herbert, Dr.**
**Breslauer Strasse 39**
**D-6233 Kelkheim(Taunus)(DE)**

(54) **Verwendung von Ethylendiamintetraessigsäure als Inhibitor der Calcium/Calmodulin abhängigen Proteinkinase II oder der Proteinkinase C.**

(57) Die vorliegende Erfindung betrifft die Verwendung von EDTA oder dessen Analoga als Inhibitoren der Calcium/Calmodulin abhängigen Proteinkinase II oder der Proteinkinase C

# Verwendung von Ethylendiamintetraessigsäure als Inhibitor der Calcium/Calmodulin abhängigen Proteinkinase II oder der Proteinkinase C

Die Calcium/Calmodulin abhängige Proteinkinase II (CCPK II) und die Proteinkinase C (PKC) sind Teile eines zellulären Signal-Übertragungsmechanismus, der rezeptorgesteuert Phosphatidylinositol (4,5)bisphosphat (PIP2) zu den second messengern Inositol (1,4,5)trisophosphat (IP3) und Diacylglycerol (DAG) abbaut. IP3 setzt Calcium aus intrazellulären Speichern frei, das an Calmodulin bindet und als Calcium/Calmodulin-Komplex die CCPK II aktiviert. DAG aktiviert direkt die PKC an der cytoplasmatischen Seite der Zellmembran. Calcium sowie bekannte Aktivatoren der PKC wie DAG oder Phorbolester können Kontraktionen glatter Muskeln auslösen, Sekretions-Vorgänge stimulieren und die Proliferation von Zellen beeinflussen (Y. Nishizuka (1986) Nature 308, 693-698, (1988) ibid. 334, 661-665). Großes Interesse besteht, spezifische, hochaffine Inhibitoren für die CCPK II und die PKC zu finden, die bei der Behandlung von Herz-Kreislauf-Erkrankungen, bei rheumatischen Prozessen, Erkrankungen des Zentralnervensystems und bei Krebs eine Rolle spielen könnten.

Für die CCPK II sind bisher keine spezifischen Inhibitoren beschrieben worden.

Bisher bekannte Inhibitoren der PKC sind mehr oder weniger unspezifisch (L. G. Garland et al. (1987) TIPS 8, (1987), 334). In K. Albert et al., Proc. Natl. Acad. Sci. USA 81, (1984), 3622-3625); F. Hucho et al., FEBS Lett. 211 (1987), 207-210; J. R. McDonald and M. P. Walsh, Biochem. J. 232, (1985), 559-567 sind edogene, in Zellen selbst vorhandene inhibitorische Protein/Peptide der PKC beschrieben. Lysosphingolipide und Sphingosine sind Lipidabbauprodukte, die in Zellmembranen vorkommen und möglicherweise auch auf die PKC inhibitorisch wirken können (Y. A. Hannun und R. M. Bell, Science 235 (1987), 670-674). Endogene niedermolekulare, wasserlösliche Inhibitoren sind bisher nicht bekannt.

Aufgabe vorliegender Erfindung ist es, neue niedermolekulare Inhibitoren der CCPK II sowie der PKC bereitzustellen.

Es wurde nun überraschenderweise gefunden, daß Ethylendiamintetraessigsäure (EDTA) und dessen Analoga hervorragende Inhibitoren dieser Kinasen sind.

Die Erfindung betrifft somit die verwendung von Ethylendiamintetraessigsäure (EDTA) oder dessen Analoga als Inhibitoren der Calcium/Calmodulin abhängigen Proteinkinase II oder der Proteinkinase C.

Bevorzugte Analoga von EDTA sind dessen Ester, insbesondere aliphatische Ester mit 1 bis 6 Kohlenstoffatomen. Ein besonders bevorzugtes Analoga ist der Ethylester.

Zur Inhibierung von CCPK II und PKC wird jedoch ganz besonders bevorzugt EDTA selbst verwendet.

EDTA und dessen Analoga hemmen die CCPK II und die PKC je nach Konzentration bis zu 100 %.

Zur Bestimmung der CCPK II-Aktivität dient eine Membranpräparation aus dem Rattengehirn, die zusammen mit markierten ATP (gamma-P32-ATP) und ohne bzw. mit Calmodulin als Aktivator inkubiert wird. Die Analytik erfolgt durch Auftrennung der durch P32-Phosphat-Übertragung radioaktiv markierten Protein in der SDS-Polyacrylamid-Gelelektrophorese, anschließendder Autoradiographie und Quantifizierung durch Laser-Densitometrie.

Zur Bestimmung der Aktivität der PKC dient eine Präparation aus dem Rattengehirn, die zusammen mit markiertem ATP (gamma-P32-ATP), Histon als Substratprotein, Phosphatidylserin, Calcium und/oder DAG als Aktivatoren inkubiert wird (t = 0,5 bzw. 1 oder 2 min.). Die Phosphorylierungsreaktion wird a) durch pipettieren auf Phosphocellulose oder b) Versetzen mit 3 % SDS (Natriumdodecylsulfat) gestoppt. Bei a) wird überschüßiges markiertes ATP durch mehrmaliges Waschen der Phosphocellulose, bei b) durch SDS-Gelelektrophorese nach Laemmli (U. K. Laemmli (1970) Nature 225, 680-688) entfernt. Bestimmung der Radioaktivität auf der Phosphocellulose (Cerenkov radiation im Beta-counter) (a)), oder Densitometrie eines über das getrocknete SDS-Elektrophorese-Gel gelegten Röntgenfilms (b)) erlaubt die Quantifizierung der PKC-Aktivität in Abwesenheit (Kontrolle = 100 %) und Gegenwart des Inhibitors.

Aufgrund der genannten Hemmwirkung können EDTA und dessen Analoga zur Behandlung von Herz-Kreislauferkrankungen (z.B. Hypertonie, Atherosklerose) Krankheiten des Zentralnervensystems, Entzündungsprozessen, Krebs und viralen Infektionen Anwendung finden.

Das folgende Beispiel erläutert die Erfindung

## a) Anreicherung und Aktivitätstest der CCPK II

Die Anreicherung und der Assay der CCPK II erfolgen im wesentlichen nach Schulman und Greengard (Schulman, H. J. and Greengard, P. (1978) Nature 271, 478-479).

## b) Reinigung der PKC

Die PKC wird aus Rindergehirn isoliert, modifiziert nach Walton (G. M. Walton et al. (1987) Anal. Biochem. 161, 425-437). Alle folgenden Schritte werden bei 4° C durchgeführt:
200-250 g Rindergehirn (Cortex) werden in 1 l Homogenisierungspuffer (20 mM Triethanolamin pH 7,4; 10 mM EGTA; 2 mM EDTA; 1 mM Dithioerythritol; 2 mM Phenylmethylsulfonylfluorid; 10 mg/ml Aprotinin; 10 mg/ml leupeptid) homogenisiert und für 1 h bei 16.000 xg zentrifugiert. Der Überstand wird durch Glaswolle filtriert und auf eine DEAE-Cellulose-Säule (180 ml Bett-Volumen equilibriert mit 20 mM Triethanolamin pH 7,4; 10 mM EGTA; 2 mM EDTA, 50 mM Beta-Mercaptoethanol) geladen. Die Säule wird zunächst mit 500 ml Puffer A (20 mM Triethanolamin pH 7,4; 1 mM EGTA; 1 mM EDTA; 10 mM Beta-Mercaptoethanol) dann mit 1000 ml 20 mM NaCl in Puffer A gewaschen. Die PKC wird mit 500 ml 90 mM NaCl in Puffer A eluiert. Das Eluat wird mit NaCl zu einer Ednkonzentration von 1 M versetzt und auf eine Phenyl-Sepharose-Säule (Bett-Volumen 50 ml, equilibriert in 1 M NaCl in Puffer B (20 mM Triethanolamin pH 7,4; 2 mM EGTA; 2 mM EDTA; 1 mM Dithioerythritol)) geladen. Die Säule wird mit 500 ml 1 M NaCl in Puffer B gewaschen und mit 250 ml eines Gradienten von 1 M NaCl bis 0 M NaCl in Puffer B eluiert. Die die PKC enthaltenden Fraktionen werden vereinigt mit Glycerol versetzt (10 %- (v/v) Endkonzentration) und auf eine Protamin-Agarose-Säule (Bett-Volumen 10 ml equilibriert in 0,1 M NaCl und 10 %(v/v) Glycerol in Puffer B) geladen. Die Säule wird mit 40 ml des Equilibrierungspuffers gewaschen und mit 120 ml eines Gradienten von 0,1 M NaCl bis 1,5 M NaCl in Puffer B und 10 % (v/v) Glycerol eluiert. Die die gereinigte PKC enthaltenden Fraktionen werden vereinigt, gegen Puffer B dialysiert mit Glycerol (Endkonzentration 10 % (v/v)) und Triton X 100 flüssigem Stickstoff und bei -70° C in Aliquots aufbewahrt. Die so erhaltene PKC ist mindestens zu 95 % rein in der SDS-Gelelektrophorese und weist eine spezifische Aktivität von 2-3 mikromol übertrages Phosphat pro min und mg auf.

## c) Aktivitätstest der PKC

α) Die Aktivität der PKC wird nach Roskoski bestimmt (R. Roskoski (1983) Methods Enzymol. 99, 3-6). Die Reaktionsmischung enthält 20 mM Triethanolamin pH 7,4; 4 mM Magnesiumacetat; 0,4 mg/ml Histon IIIS, 50 mM Beta-Mercaptoethanol, 20 mikroM ATP mit Spuren von gamma-P32 markiertem ATP (spezifische Aktivität 2-10 x 10 exp6 cpm/nmol); 1-100 nM PKC und unter stimulierten Bedingungen 0,1 mg/ml Phosphatdidylserin (in vesikulärer Form) zusammen mit 1 mikrogramm/ml phorbol 12-myristat 13-acetat. Freies Calcium wird mit einem Ca/EGTA-Puffer auf 0,1 bis 100 mikromolar eingestellt.

β) Alternativ: Die Reaktionsmischung enthält 50 mM Hepespuffer pH 7,4; 10 mM Magnesiumchlorid; 1 mM EGTA; 2 mM Dithioerythritol, 0,1 mg/ml Histon IIIS, 10 mikroM gamma-P32-ATP (spez. Aktivität 10-20 x 10 exp7 cpm/nmol); 1-100 nM PKC und unter stimulierten Bedingungen 0,05 mg/ml vesikuläres Phosphatdidylserin zusammen mit 0,1 mM freiem Calcium.

Die Reaktion wird normalerweise durch Zugabe des markierten ATP gestartet, bei kinetischen Experimenten durch Zugabe der PKC. Reaktionszeiten liegen je nach Versuch zwischen 0,5 und 10 min bei 30° C, bevorzugt bei 1 min. Das Volumen der Reaktionsmischung liegt je nach Versuch zwischen 40 und 100 mikrolietern, bevorzugt 80 mikroliter. Die Quantifizierung erfolgt durch Pipettieren eines Aliquots (30 oder 40 mikroliter) der Reaktionsmischung nach den angegebenen Zeiten auf Phosphocellulose (P 81, Whatma), Waschen der Phosocellulose (drei mal mit 0,05 % Phosphorsäure) und BEstimmung der Radioaktivität des gebunden phosphorylierten Histons durch Cerenkov-Strahlung im Beta-Counter. Alternativ kann die Reaktion mit 2 % (w/v) SDS gestoppt und einer Gelektrophorese nach Laemmli (U. K. Laemmli (1970) Nature 227, 680-685) unterzogen werden. Nach Trocknen des Gels und Autoradiographie wird die Schwärzung durch Laser-Densitometrie quantifiziert.

## d) Bestimmung der Aktivität der cAMP-abhängigen Proteinkinase

Die Aktivität der katalytischen Untereinheit der cAMP-abhängigen Proteinkinase wird nach Roskoski gemessen (Roskoski, R. (1983) Methods Enzymol. 99, 3-6).

## e) Spezifität der Hemmung

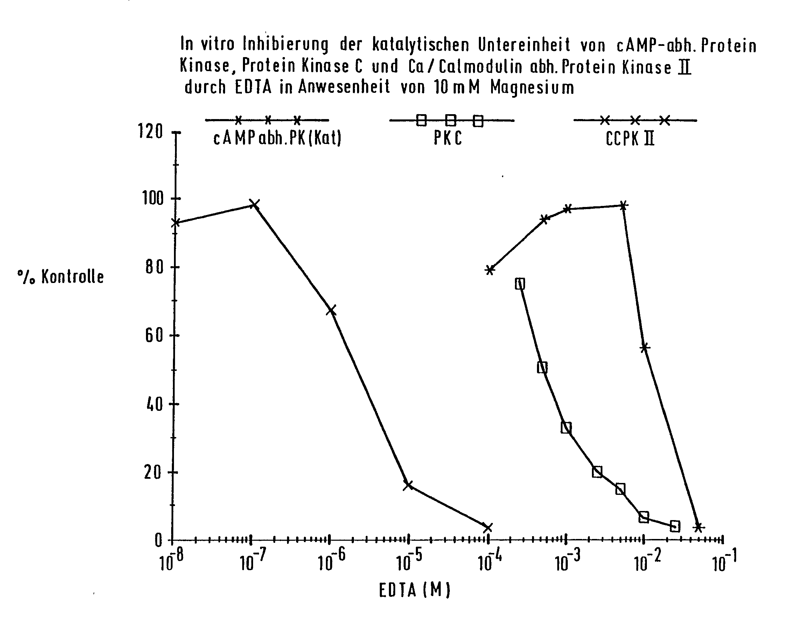

Figur 1 zeigt, daß EDTA die CCPK II mit einer halbmaximalen Konzentration von 2 mikroM, die PKC mit einer halbmaximalen Konzentration von 400 mikroM inhibiert. Die halbmaximale Hemmkonzentration von EDTA für die katalytische Untereinheit der cAMP-abh. Proteinkinase liegt bei 10 mM.

Eine Hemmkonzentration von 10 mM, wie bei der cAMP-abh. Proteinkinase gefunden, entspricht der in allen Testen vorhandenen Konzentration von Magnesium-Ionen. Deren vollständige Komplexierung durch EDTA führt zur unspezifischen Hemmung aller Magnesium-ATP-abhängigen Prozesse.

Die Hemmung der CCPK II und der PKC durch EDTA muß jedoch auf einem anderen Mechanismus beruhen, da hier ebenfalls freie Magnesium-Ionen in einer Konzentration von 10 mM vorlagen.

Eine freie Calcium-Ionen-Konzentration von 1 mM in den CCPK II- und PKC-Assays liegt ebenfalls deutlich über den gefundenen halbmaximalen Hemmwerten von EDTA, so daß auch eine Komplexierung von Calcium-Ionen als Hemmechanismus nicht in Frage kommt.

**Ansprüche**

1. Verwendung von Ethylendiamintetraessigsäure (EDTA) oder dessen Analoga zur Herstellung eines Mittels zur Inhibierung der Calcium/Calmodulin abhängigen Proteinkinase II oder der Proteinkinase C.

2. Verwendung nach Anspruch 1, dadurch gekennzeichnet, daß das Mittel zur Behandlung von Herz-Kreislauf-Erkrankungen, Krankheiten des Zentralnervensystems, Entzündungsprozessen, Krebs und viralen Infektionen geeignet ist.

In vitro Inhibierung der katalytischen Untereinheit von cAMP-abh. Protein Kinase, Protein Kinase C und Ca/Calmodulin abh. Protein Kinase II durch EDTA in Anwesenheit von 10 mM Magnesium

EP 0 368 223 A2